# EUROPÄISCHE PATENTSCHRIFT

(11) **EP 1 185 310 B1**
(45) Veröffentlichungstag und Bekanntmachung des Hinweises auf die Patenterteilung: **03.09.2003**
(21) Anmeldenummer: 00949133.3
(22) Anmeldetag: 23.06.2000
(51) Int. Cl.: A61L 9/12, A63J 23/02, A44C 15/00

(54) **GERÄT ZUR ABGABE VON DÜFTEN UND AROMENSPEICHER (DUFTCHIP)**
APPLIANCE FOR DISPENSING SCENTS AND AN AROMA STORE (SCENT CHIP)
APPAREIL POUR DIFFUSER DES SENTEURS ET RESERVOIR DE SENTEURS (PLAQUETTE DE SENTEURS)

(30) Priorität: 22.06.1999 DE 19928592; 17.04.2000 DE 10018914
(43) Veröffentlichungstag der Anmeldung: 13.03.2002
(73) Patentinhaber: Stefan Ruetz Technologies, 80807 München (DE)
(72) Erfinder: RUETZ, Stefan, D-80639 München (DE); PROBST, Gerhard, D-81247 München (DE); SCHATZ, Peter, D-85757 Karlsfeld (DE); BREU, Christian, D-85748 Garching (DE); WAGENSONNER, Heinz, D-84101 Obersüssbach (DE)
(74) Vertreter: Zipse & Habersack
(86) Internationale Anmeldenummer: DE0002081
(87) Internationale Veröffentlichungsnummer: WO00078367

(56) Entgegenhaltungen:
- EP-A- 0 674 468
- EP-A- 0 831 384
- DE-A- 19 715 404
- US-A- 4 629 604
- "SCENT GENERATOR" IBM TECHNICAL DISCLOSURE BULLETIN,US,IBM CORP. NEW YORK, Bd. 38, Nr. 11, 1. November 1995 (1995-11-01), Seite 429 XP000547415 ISSN: 0018-8689

## Beschreibung

Die Erfindung betrifft ein Gerät zur Abgabe von Düften gemäß dem Oberbegriff des Anspruchs 1, sowie einen Aromenspeicher (Duftchip) insbesondere zur Verwendung mit einem solchen Gerät zur Abgabe von Düften.

Aus EP 0 611 476 B1 ist ein Verfahren zur Erhöhung der sinnlichen Wahrnehmung von visuellen und/oder akustischen Darbietungen in Kino-, Theater- oder Konzertsälen bekannt, wobei Zuschauern bzw. Zuhörern synchron zur Darbietung von bestimmten visuellen und/oder akustischen Ereignissen bzw. Szenen dazu passende Düfte zugeführt werden. Die Zuführung der Düfte erfolgt mittels des Trägergases Luft. Hierzu sind zu den Sitzen des Vorführsaales miniaturisierte Einzelleitungen verlegt mit Luftauslässen, beispielsweise in den Armstützen oder in den Rückenlehnen der Vordersitze. Die Duftvorräte liegen in freigebbarer fester oder flüssiger Form vor und sie werden durch Kontaktierung an einen durchströmenden Luftstrom abgegeben, der von einer Druckluftquelle in die Einzelleitungen abgegeben wird. Die beim Zuschauer oder Zuhörer insgesamt austretende Luftmenge, welche den Duft transportiert, soll weniger als ein Liter/sec und vorzugsweise zwischen 0,3 und 0,00001 Liter/sec betragen.

Ein solches System zur begleitenden Beduftung von Filmvorführungen, Musikdarbietungen oder Theateraufführungen ist schwierig am Markt durchsetzbar, da es aufwendige Installationen in den betreffenden Sälen erfordert, und die Kosten für solche Investitionen erst verantwortbar sind, wenn ausreichend Produktionen am Markt sind. Umgekehrt wird man Produktionen mit Duftbegleitung erst in Angriff nehmen, wenn ausreichend umgerüstete Säle zur Verfügung stehen.

EP 0 732 132 A2, eine Ausscheidung aus EP 0 611 476 B1, stellt ein entsprechendes duftbegleitendes System mit Luft als Trägergas vor, das auf feste Installationen von Leitungen verzichtet und die Düfte den Zuschauern bzw. den Zuhörern synchron zur Darbietung von bestimmten visuellen und/oder akustischen Ereignissen bzw. Szenen aus einer mobilen Einheit zuführt. Die mobile Einheit besitzt einen sogenannten Duftcomposer, in dem die benötigten Duftnoten aus Duftgrundkomponenten in Duftmischrollen zusammengemischt werden. Mit einem kleinen, innerhalb des Duftcomposers untergebrachten Tangentialgebläse wird Luft erzeugt, die beim Durchfluß durch die Duftmischrollen den speziellen Duft der Duftgrundkomponenten aufnimmt, um daraus den benötigten Duft zu mischen. Der Duftcomposer soll sich für die Verwendung in kleineren Räumen für dezentrale Anwendungen eignen, wie bei Diavorträgen oder für die Ausstrahlung von Video- und Fernsehsendungen. Es wäre dabei möglich, die Signalleitung zur Mischrollensteuerung an ein Videogerät oder einen Fernseher anzuschließen, wobei der zu bestimmten Szenen zugehörige Regelimpuls dann über ein Funksignal an den Fernseher gesendet wird.

Die DE 197 15 404 A betrifft eine als Duftspender ausgebildete Vorrichtung mit mehreren Duftstoffspeichern, wobei eine Auswahleinrichtung vorgesehen ist, mit welcher ein bestimmter Duftstoffspeicher angesteuert werden kann, um eine vorgebbare Menge des in ihm vorhandenen Duftstoffs abzugeben. Bevorzugt wird ein Treibgas zum Ausbringen des Duftstoffes verwendet. Das Ausbringen der Duftstoffe kann z.B. mit einem Videofilm synchronisiert werden.

Der Erfindung liegt die Aufgabe zugrunde, ein Gerät zur Abgabe von Düften zu schaffen, mit dem es möglich ist, ereignis- oder szenenbezogen dazu passende Düfte abzugeben, das ohne Installationsaufwand individuell einsetzbar ist, mit geringsten Mengen an Aroma- bzw. Duftstoffen auskommt und hygienischen Ansprüchen genügt. Das zu schaffende Gerät soll sich auch ganz allgemein zur Abgabe von einzelnen oder einer Folge von Düften eignen, wobei die Eingabe für den bzw. die auszutragenden Düfte manuell oder per Signalübertragung, z. B. auch über Internet erfolgen kann. Der Erfindung liegt auch die Aufgabe zugrunde, einen Aromenspeicher (Duftchip) zu schaffen, der äußerst preiswert als Massenartikel herstellbar und mit einem Gerät zur Abgabe von Düften verwendbar ist.

Diese Aufgabe wird erfindungsgemäß mit einem Gerät zur Abgabe von Düften gelöst, wie es durch den Anspruch 1 gekennzeichnet ist. Weiterbildungen der Erfindung sind in den Unteransprüchen beschrieben. Ein Aromenspeicher (Duftchip) nach der Erfindung ist durch den Anspruch 27 und durch die auf ihn zurückbezogenen Unteransprüche gekennzeichnet.

Das Gerät nach der Erfindung zeichnet sich unter anderen dadurch aus, daß zum Austragen der Düfte kein Trägergas wie zum Beispiel Luft verwendet wird, sondern dieser Austrag unmittelbar aus einem Aromenspeicher, beispielsweise mittels einer Mikrodosierpumpe und Zerstäuben oder durch Verdampfen erfolgt. Die auf diese Weise generierte Duft- oder Aromenwolke steigt durch natürliche Konvektion aufgrund der Körperwärme zur Nase des Nutzers. Ihre Intensität liegt oberhalb der Wahrnehmungsgrenze und sie löst damit den gewünschten olfaktorischen Reiz aus.

Das Gerät wird am Körper des Nutzers getragen und wird vor der Brust wie eine Brosche angebracht, wie eine Kette umgehängt oder wie ein Kugelschreiber in die äußere Brusttasche gesteckt. Es ist auch möglich, das Gerät in sonstiger Weise in großer Nähe des Nutzers anzuordnen, indem es beispielsweise an eine Armstütze (oder an einem ausklappbaren Arm) eines Sessels befestigt wird. Durch die Nähe zur Nase kann mit geringsten Duft- und Aromenmengen gearbeitet werden. Zur Unterstützung der Aufwärtsbewegung der Duft- oder Aromenwolke aufgrund natürlicher Konvektion kann dem Gerät ein kleines Gebläse und eventuell auch eine Heizeinrichtung zugeordnet werden. Durch die Wirkung der Heizeinrichtung gelingt es auch, daß sich manche Düfte voller entfalten.

Nach der Wahrnehmung verflüchtigt sich die Duft-/Aromenwolke durch Vermischung mit der Umgebungsluft. Die Intensität der Duft-/Aromenwolke fällt dabei rasch unter die Wahrnehmungsgrenze. Zusätzlich wird durch das Phänomen der olfaktorischen Adaption der Sinnesreiz beendet. Dadurch wird die Erzeugung von zeitlich exakt eingegrenzten Riecherlebnissen ermöglicht.

Eine Steuereinheit, zweckmäßig mit einem Empfangsmodul zur externen Ansteuerung durch eine Signal- oder Taktgebereinheit, ermöglicht eine Abgabe passender Düfte synchron zur jeweiligen Darbietung. Am Einsatzort sind keine komplexen Installationsoder Umbauarbeiten nötig, lediglich eine zusätzliche Signaloder Taktgebereinheit wird vor Ort installiert. Die Geräte selbst haben eine unabhängige Stromversorgung durch Batterie oder Akku. Die Düfte und Aromen werden einzeln und direkt aus den Speichern unmittelbar in die Umgebungsluft ausgebracht, ohne Durchleiten durch gemeinsame Leitungen und Düsen, wodurch die Notwendigkeit zusätzlicher Reinigungsprozeduren entfällt. Systembedingt ist keine Druckluftunterstützung notwendig, weshalb es keine Probleme mit Störgeräuschen gibt. Durch die Miniaturisierung kann das Gerät in Form eines unauffälligen oder auch attraktiven Accessoires vom Nutzer getragen oder in seiner unmittelbaren Nähe montiert, angeklemmt oder angeklipst werden.

Das Gerät nach der Erfindung ist sowohl für den privaten als auch professionellen Einsatz bestimmt, als neue Dimension nicht nur bei medialen Anwendungen. Anwendungsbeispiele sind: Shopping, Entspannung, Meditation, Videospiele, Fernsehen, Video, Computersimulationen, Internet, Kino, Theater, Ausstellungen, um nur einige zu nennen.

Eine Möglichkeit, die Duft-/Aromenwolke und damit auch die Duftspeichermenge gering zu halten, besteht darin, den Duft synchron zum Atmungsvorgang des Nutzers auszutragen, dessen Zyklus etwa 6-8 Sekunden beträgt. Der Duft wird unter Einberechnung der Duftfreisetzungszeit und der durchschnittlichen Strömungszeit des Duftes zur Nase nur ausgetragen, wenn er während eines Einatmungsvorgangs zur Nase gelangen kann. Diese Kopplung an den Atmungsvorgang kann auch genutzt werden, um z. B. einen Einschlafzustand des Nutzers festzustellen, um dann einen "Aufweck-" Duft auszustoßen.

Die Erfindung wird nachfolgend anhand beigefügter Zeichnungen näher beschrieben. Es zeigen
Figuren 1a und b schematisch das Gerät nach der Erfindung im professionellen Einsatz am Beispiel Kino bzw. im privaten Einsatz am Beispiel Fernsehen oder Internet;
Figur 2 den schematischen Aufbau eines Geräts nach der Erfindung;
Figur 3 die Anbringung des Geräts nach der Erfindung vor der Brust eines Nutzers;
Figur 4 schematisch den Aufbau einer Aromenvorratskartusche;
Figur 5 schematisch den Aufbau eines Aromenspeicherchips mit vergrößerten Darstellungen eines Speicherplatzes
   a) im Ruhezustand,
   b) beim Aufheizen und
   c) beim Austrag einer Aromenwolke;
Figur 6 schematisch bevorzugte Ausführungsvarianten eines Aromenspeichers (Duftchips) unter Verwendung von Porösstoffen als Duftstoffträger
   a) mit Erwärmung mittels IR-Laser,
   b) mit Erwärmung mittels Widerstands- Heizelement,
   c) und d) mit Isolierung des Porösstoffes von der Trägerfolie mittels Aluschale bzw. alubedampfter Kunststoffschale;
   e) Einbettung des Porösstoffes in einen Harz- oder Kunststoffträger und
   f) Einbettung des Porösstoffes in die wärmeleitende Masse;

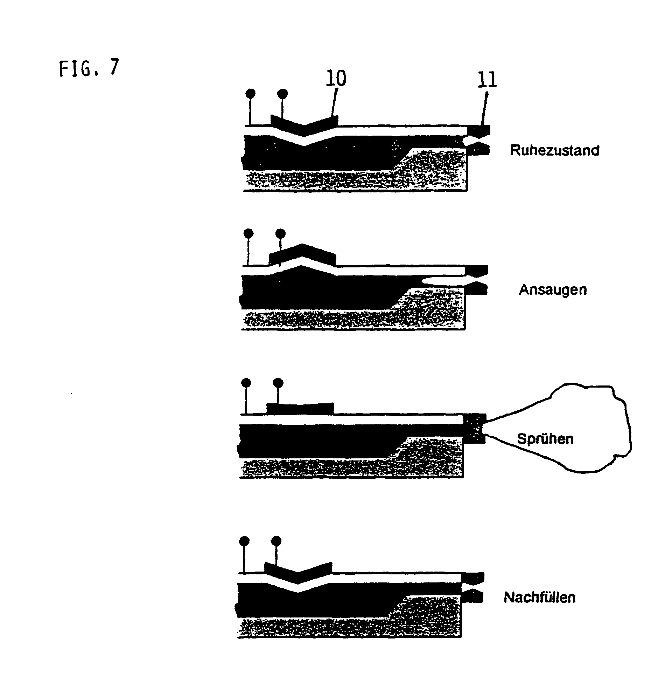

Figur 7 schematisch in vergrößerter Darstellung eine Mikrodosierpumpe auf der Basis piezoelektrischer Aktoren mit mechanischer Zerstäubungsdüse;

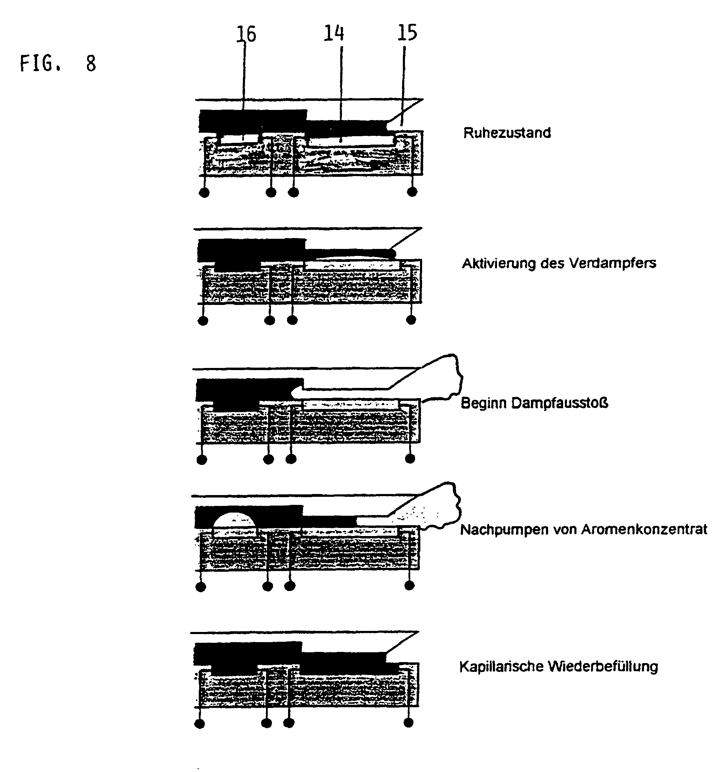

Figur 8 schematisch in vergrößerter Darstellung eine Mikrodosierpumpe auf der Basis thermischer Aktoren mit einem Mikroheizelement zum Verdampfen der ausgetragenen Duftstoffe;

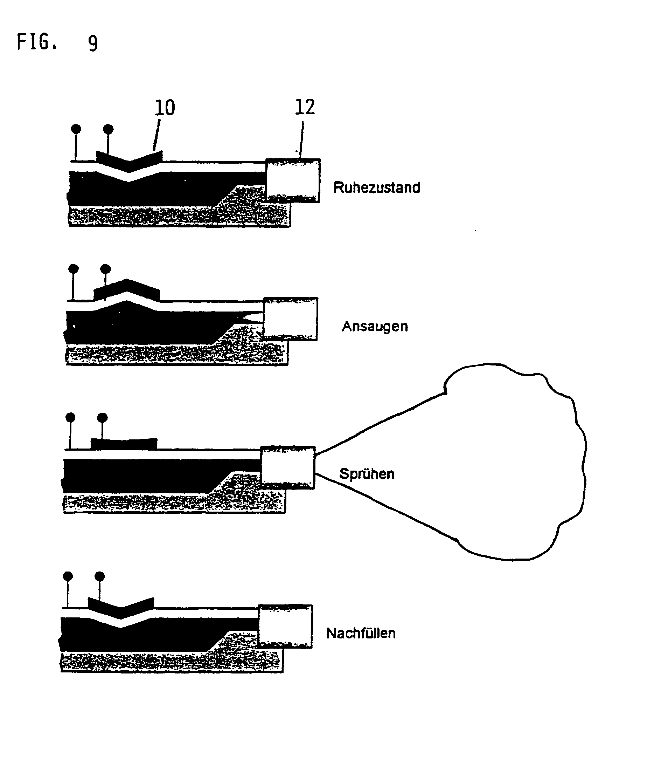

Figur 9 schematisch in vergrößerter Darstellung eine Mikrodosierpumpe auf der Basis piezoelektrischer Aktoren mit Ultraschallzerstäubung;

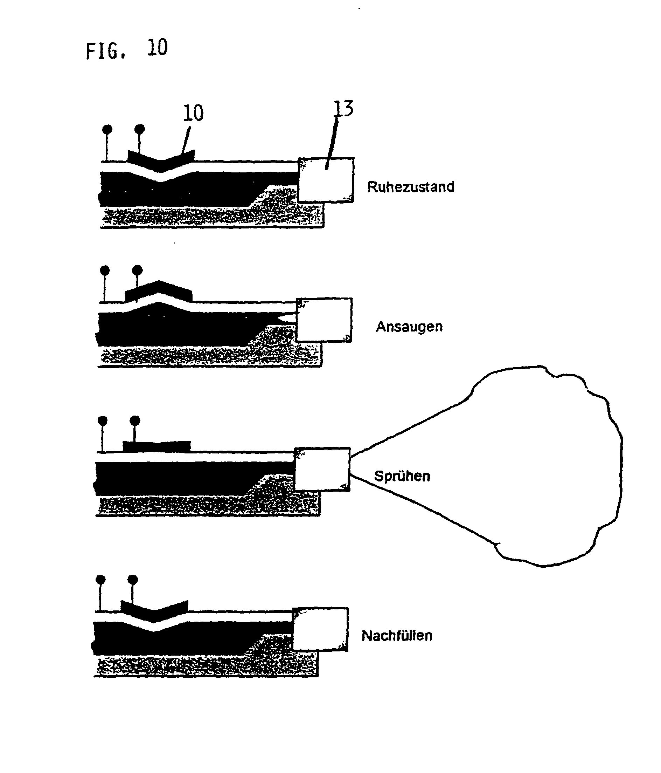

Figur 10 schematisch in vergrößerter Darstellung eine Mikrodosierpumpe auf der Basis piezoelektrischer Aktoren mit elektrostatischer Zerstäubung;

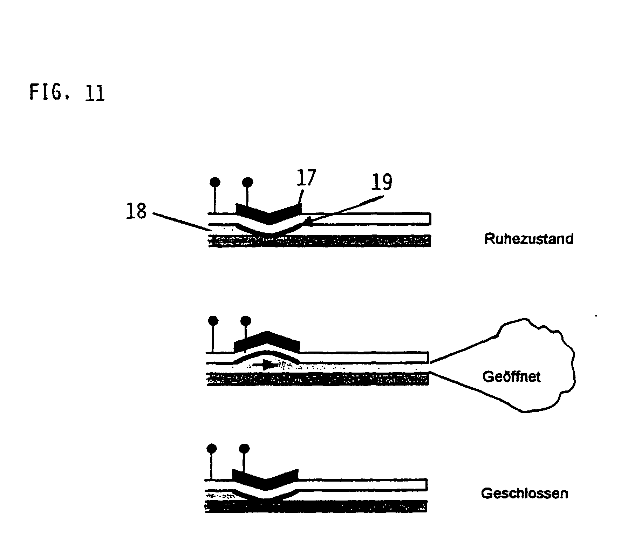

Figur 11 schematisch in vergrößerter Darstellung den Austrag gasförmigen Aromenkonzentrats mit Piezoventil-Steuerung;

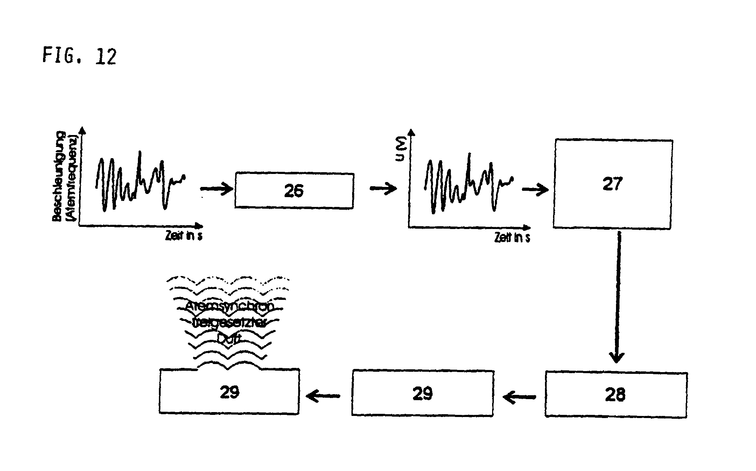

Figur 12 eine Schemadarstellung einer atemsynchronen Duftfreisetzung.

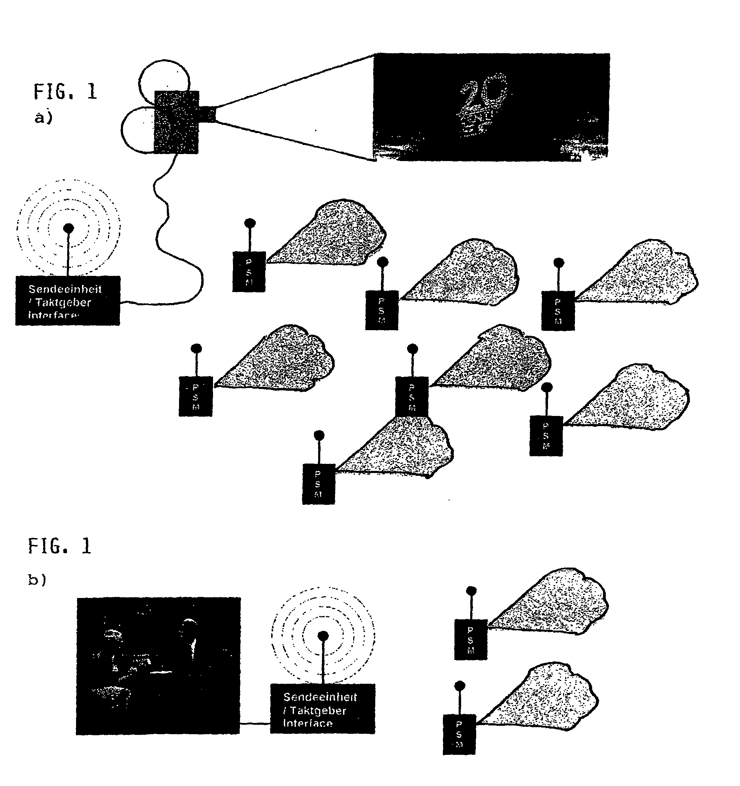

In Figur 1a und b wird das Gerät nach der Erfindung im professionellen Einsatz am Beispiel Kino bzw. im privaten Einsatz am Beispiel Fernsehen bzw. Internet gezeigt. Das Gesamtsystem besteht aus einer stationären Sendeeinheit und einer beliebigen Anzahl erfindungsgemäßer Geräte, jeweils ausgerüstet mit einem Empfangsmodul. Die Sendeeinheit stellt zusätzlich das Taktgeberinterface dar, d. h. die Sendeeinheit wird mit entsprechenden Vorführgeräten oder Taktgebereinheiten verbunden. Zur Ansteuerung der Duft-/Aromensequenzen werden Signale wie z. B. Timecode oder ähnliches verwendet. Die Geräte nach der Erfindung können aber auch in einem Stand-alone-Modus ohne externe Ansteuerung betrieben werden.

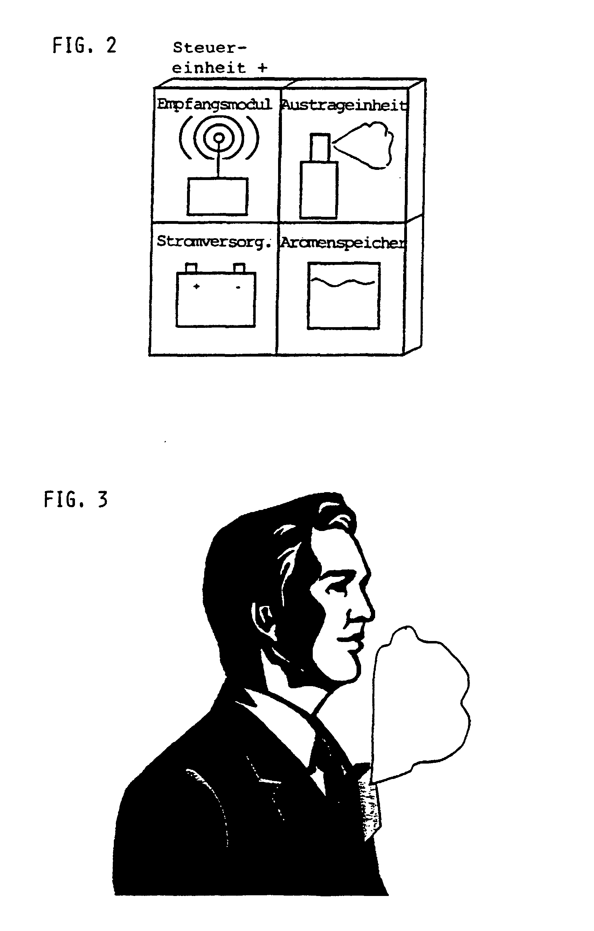

Das Gerät selbst stellt ein mobiles System zur Erzeugung von Riecherlebnissen dar. Es besteht gemäß Figur 2 im wesentlichen aus vier verschiedenen Baugruppen, der Steuereinheit mit Empfangsmodul, der Stromversorgung (Energiespeicher), dem Aromenspeicher und der Austrageinheit zur Generierung und zum Austragen einer Duft- oder Aromenwolke. Durch Miniaturisierung kann das Gerät in etwa die Größe einer Brosche oder eines Kugelschreibers haben und gemäß Figur 3 am Körper des Nutzers getragen werden.

Das Aromenkonzentrat kann im Aromenspeicher in gasförmiger, flüssiger, fester (pastöser) Form oder als Gel gespeichert werden. Der Austrag und die Generierung einer Duft- oder Aromenwolke erfolgt je nach Art der Speicherung des Aromenkonzentrats ohne Zuhilfenahme eines Trägergases z. B. durch Verdampfen mittels Mikroheizelementen bzw. Laserenergie oder mittels Mikrodosierpumpe, durch Ventilsteuerung, wie anhand nachfolgender Zeichnungen näher erläutert wird.

Das Herzstück des Geräts nach der Erfindung sind der Aromenspeicher und die Austrageinheit zum Generieren und Austragen einer Duft- oder Aromenwolke.

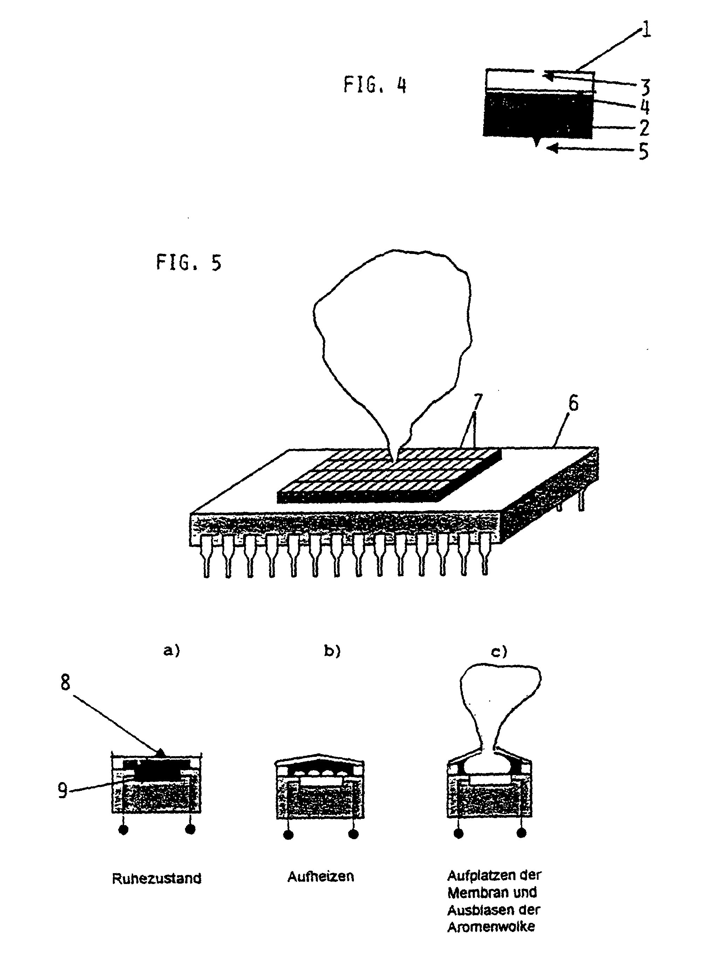

Der Aromenspeicher kann gemäß Figur 4 eine Aromenvorratskartusche sein, die in vielen Einzelkammern 1 die benötigten Aromenkonzentrate 2 speichert. Die Einzelkammern 1 können je nach Anwendung mit gleichen oder unterschiedlichen Aromenkonzentraten befüllt werden. Um ein Nachfließen des Aromenkonzentrats beim Betrieb zu gewährleisten ist an der Oberseite der Einzelkammern 1 eine Entlüftungsöffnung 3 angebracht. Desweiteren wird innerhalb jeder Einzelkammer 1 durch eine Schutzmembran oder -folie 4 ein unerwünschtes Austreten von Aromen verhindert. Zur Ansteuerung kann an die Einzelkammern der Kartusche eine elektronisch auslesbare Kennung angebracht sein, die Auskunft über Duftart, Konzentration und Aufbereitung gibt. Die Abgabe des Aromenkonzentrats erfolgt durch eine Austrittsöffnung 5.

Eine Aromenvorratskartusche dient insbesondere der Aufbewahrung von Aromenkonzentraten, wenn diese in flüssiger Form vorliegen. Bei gasförmiger Aufbewahrung bilden die Einzelkammern Druckkammern, wobei eine Entlüftungsöffnung und eine Schutzmembran entfallen. Die Austrittsöffnung muß durch eine aufbrechbare Öffnung oder durch ein Steuerventil verschlossen sein.

Vorzugsweise kommt ein Aromenspeicher in Form eines Mikrochip oder als Chipkarte (Duftchip) mit Duftstoffe- Speicherplätzen z. B. gemäß Figur 5 in Frage. Die Aromen werden im Chip 6 bzw. auf einem Träger in kleinen Mikrokammern oder Mikrotanks 7 oder auf kleinen Speicherplätzen flüssig, als Feststoff, als Gel, oder auch als Gas gespeichert. Insbesondere im Falle der flüssigen/gasförmigen Speicherung oder als Gel sind die Aromen oder Duftstoffe geschützt unter einer Schutzschicht oder -folie 8 untergebracht. In dem Gerät ist den Duftstoff-Speicherplätzen jeweils ein von der Steuereinheit ansteuerbares Element zum z. B. thermischen und/oder elektro-chemischen Austragen von Duftstoff zugeordnet. Durch Erhitzen eines unter der Kammer 7 bzw. dem Speicherplatz befindlichen Heizelements 9 wird das Aromenkonzentrat verdampft und durch den dabei entstehenden Druck wird die Schutzfolie 8 zum Platzen gebracht. Ein Austreten von Aromenkonzentrat in Tropfenform kann durch eine gasdurchlässige Membran (Goretex- eingetragene Marke) verhindert werden. Durch den Verdampfungsvorgang wird gleichzeitig mit dem Aromenaustrag eine Duft- bzw. Aromenwolke generiert. Insbesondere mit aufplatzender Schutzfolie 8 dienen die Aromenspeicherplätze 7 dem einmaligen Gebrauch, d. h. nach Ansteuerung und Verdampfen des Speicherplatzes ist das dort vorhandene Aromenkonzentrat im wesentlichen verbraucht. Für Düfte, die häufiger benötigt werden, sind dann entsprechend viele Speicherplätze mit demselben Aromenkonzentrat zu belegen. Zur Steigerung der Intensität eines Duftes können gleichzeitig mehrere Speicherplätze 7 aktiviert werden. Nach Ende einer Darbietung wird im allgemeinen der Chip bzw. die Chipkarte ausgewechselt.

Es ist auch möglich, den Duftstoff-Speicherplätzen ein Reagenz zuzuzuordnen, zur Auslösung einer z. B. exothermen Reaktion unter definierten Bedingungen. In dem Gerät ist dann den Duftstoff-Speicherplätzen jeweils ein von der Steuereinheit ansteuerbares Element zur Schaffung dieser definierten Bedingungen für das Reagenz zugeordnet.

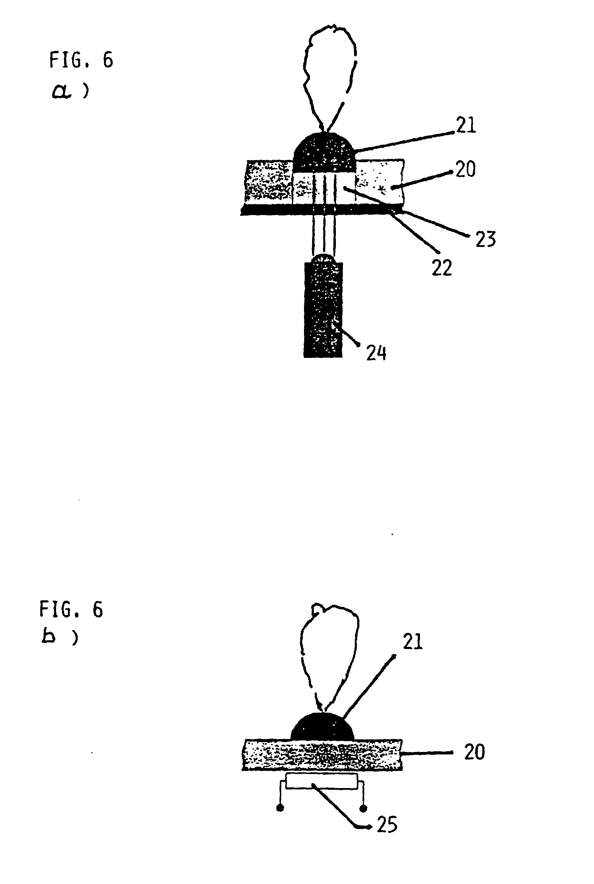

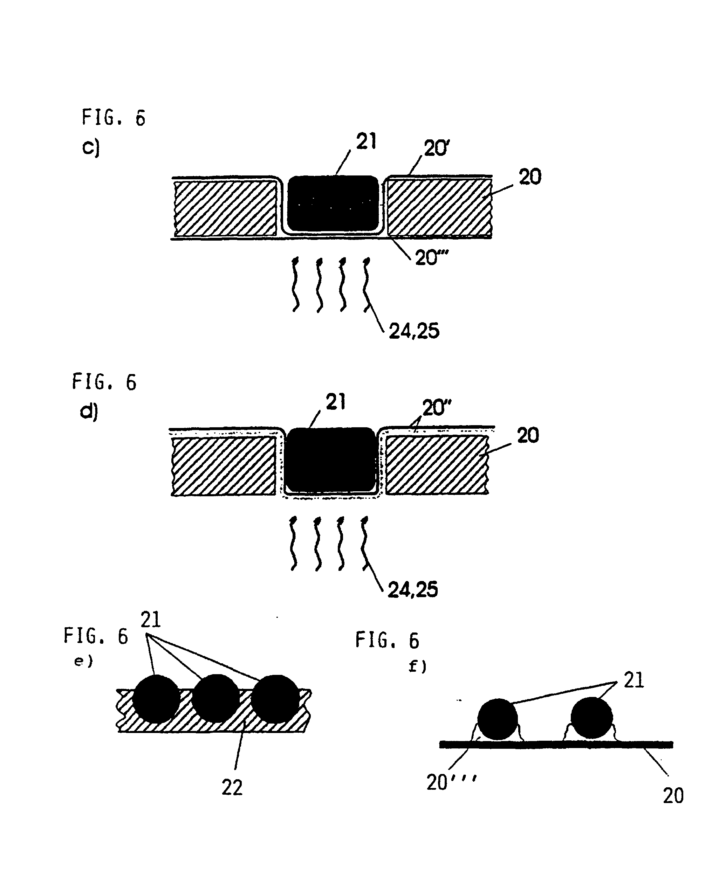

In Figur 6 sind bevorzugte Ausführungsvarianten eines Duftchips dargestellt, die mit einem Porösstoff (wie Zeolith) als Träger fur den flüssigen, gelförmigen oder festen Duftstoff und mit thermischer Freisetzung des Duftstoffs arbeiten. Auf bzw. in eine kostengünstige Duftträgerfolie 20 (z. B. Harz/Kunststoffolie wie PTFE-Folie oder Pappe) ist Porösstoff 21 schachbrettartig in geringer Menge auf- bzw. eingebracht.

Im Falle der Figur 6 a) ist die Duftträgerfolie 20 perforiert, wobei der Porösstoff 21 nach Art eines Stöpsels das jeweilige Perforationsloch nach oben verschließt. Auf ihrer Unterseite ist die perforierte Folie 20 mit einer dünnen Folie 22 versiegelt. Die dabei gebildeten Hohlräume 23 dienen als Reservoir für die unterschiedlichen flüssigen Duftstoffe, die ihrerseits in den Porösstoff diffundieren. Um unerwünschtes "Abduften" zu verhindern, können die Porösstoffe 21 an ihrer Oberseite z. B. mittels Wachs versiegelt sein. Unterhalb des Duftträgers 20-23 sind im Gerät auf einer hochwärmeleitenden Al₂O₃ Keramik Hochleistungs-IR-Laser 24 (VCSEL) in einer entsprechenden schachbrettförmigen Struktur angebracht. Die einzelnen Lasereinheiten haben eine Größe von etwa 0,35 x 0,35 mm. Über eine in Siebdrucktechnik hergestellte Schaltung können die Lasereinheiten einzeln angesteuert werden.

Im Falle der Figur 6 b) sind die einzelnen Porösstoffportionen 21 mit den unterschiedlichen Duftstoffen getränkt oder mit Duftstoffen in Form von Feststoffablagerungen bedeckt. Unterhalb der dünnen Duftträgerfolie 20 sind im Gerät auf eine Keramik- oder Stahlsubstratplatte voneinander getrennt ansteuerbare Widerstände 25 mittels Siebdrucktechnik aufgebracht. Diese ca. 0,5 x 0,5 mm großen Widerstände werden schachbrettartig über AgPb-Leitungen einzeln angesprochen und elektrisch erhitzt.

In Figuren 6 c) und d) sitzen die Porösstoffe 21 wie in Figur 6a in Perforationslöchern der Trägerfolie 20. Um zu vermeiden, dass die im Porösstoff aufgesaugten Duftstoffe in die Trägerfolie 20 diffundieren, und auch zum Bündeln der Heizenergie ist der Porösstoff in einer Aluminiumschale 20' (Figur 6c) bzw. in einer alubedampften Kunststoffschale 20'' (Figur 6d) eingebettet. Im Falle der Figur 6c ist eine elektrische Trennschicht 20''', z. B. eine Lackschicht gegen die Unterseite der Trägerfolie 20 angebracht, um die elektrisch leitende Aluminiumschale 20' elektrisch von den darunter angebrachten Heizelementen 24, 25 zu trennen. Bei entsprechender Stabilität der Aluminium- oder Kunststoffschale 20' bzw. 20'' kann auf die Trägerfolie 20 verzichtet werden.

Im Falle der Figur 6 e) sind die Porösstoffportionen in einen Harz-/Kunststoffträger 20 und im Falle der Figur 6 f) in eine wärmeleitende Masse 20''' wie z. B. in Klebesilikon mit Al₂O₃ (Bauxit) eingebettet.

### Das Funktionsprinzip ist wie folgt:

Im Falle der Figur 6 a) emittiert die IR-Laserdiode gebündelte Lichtenergie. Diese wird vom Porösstoff im Duftträgersystem absorbiert und in Wärme umgewandelt. Diese eingebrachte thermische Energie erwärmt den Porösstoff mit Duftstoff, bis der Duftstoff verdampft und somit Duftmoleküle freigesetzt werden. Durch Variation der Laserleistung und Heizdauer ist eine Dosierung der Duftstoffmenge sowie eine Mehrfachausbringung von Duftstoffen möglich.

Bei Figur 6b wird das Heizelement erwärmt und es gibt dann an das auf Kontakt anliegende Duftträgersystem Energie in Form von Wärme weiter. Diese eingebrachte thermische Energie erwärmt den Porösstoff mit Duftstoff, bis der Duftstoff verdampft und somit Duftmoleküle freigesetzt werden. Durch Variation der Heizdauer und -leistung ist eine Dosierung der Duftstoffmenge sowie eine Mehrfachausbringung von Duftstoffen möglich.

Ein Aromenspeicher (Duftchip) insbesondere gemäß Figur 6 ist äußerst preiswert als Massenartikel herstellbar. Er kann auf eine Größe von etwa 30x40 mm bei einer Dicke von etwa 1mm miniaturisiert und damit leicht z. B. in Programmzeitschriften verschickt werden. Der Nutzer muß dann lediglich den passenden Chip in sein Gerät zur Abgabe von Düften einsetzen, um bei Empfang des ensprechenden Programms (oder auch eines Werbespots) in den Genuß des Dufterlebnisses zu kommen. Auf dem beschriebenen Duftchip können beispielsweise 100 bis 400 Porösstoff- und damit Duftstoffportionen untergebracht werden, wobei je Porösstoffportion eine Saugfähigkeit von 0,1 - 0,3 µl Duftstoff für ein Riechergebnis von ca. 10 sec Dauer ausreicht.

Bei Speicherung der Düfte in flüssiger Form in einer Aromenvorratskartusche ist eine Austrageinheit erforderlich zur Generierung und zum Austrag der Duft- bzw. Aromenwolke. Eine solche Austrageinheit kann unter Zuhilfenahme verschiedenster Technologien aufgebaut sein. Im wesentlichen besteht die Austrageinheit aus zwei Funktionsbaugruppen, nämlich einer Pumpeinheit (nicht erforderlich bei gasförmiger Speicherung) und einer Zerstäubungs- oder Verdampfungseinheit.

Für die Realisierung der Pumpeinheit kommt die Verwendung von Mikroheiz- oder Piezoelementen in Frage. Die Zerstäubungseinheit kann herkömmlich aus einer mechanischen Düse bestehen oder unter Zuhilfenahme von Elektrostatik oder Ultraschall verwirklicht sein. Die Verdampfungseinheit wird mit einem Mikroheizelement realisiert. Das Leerlaufen der Austrageinheit wird durch die starken Kapillarkräfte in den Düsen verhindert. Wenn das Gerät ausgeschaltet ist, können die Düsen mechanisch mit einer Kappe abgedeckt werden, um ein langsames Austreten von Aromen zu verhindern.

Nachfolgend werden einige Austrageinheiten für flüssige Aromenkonzentrate anhand der Figuren 7 bis 11 erläutert. Figur 7 zeigt für den Austrag flüssigen Aromenkonzentrats eine Mikrodosierpumpe auf der Basis eines Piezoelements 10. Mit diesem Piezoelement 10 wird das Aromenkonzentrat mechanisch gepumpt. Bei Anlegen eines Spannungsimpulses schnellt das Piezoelement 10 nach oben und saugt Flüssigkeit an. Danach kehrt das Piezoelement in seine ursprüngliche Position zurück und pumpt dabei das Aromenkonzentrat durch eine mechanische Zerstäubungsdüse 11.

Die Figuren 9 und 10 zeigen entsprechende Mikrodosierpumpen auf der Basis eines Piezoelements 10. Anstelle einer mechanischen Zerstäubungsdüse 11 ist in Figur 9 eine Ultraschallzerstäubung 12 bzw. eine elektrostatische Zerstäubung 13 angedeutet. Es handelt sich bei der Ultraschall- bzw. elektrostatischen Zerstäubung um bekannte Technologien, so dass sich eine nähere Beschreibung erübrigt.

Figur 8 zeigt den Austrag flüssigen Aromenkonzentrats mittels einer Mikrodosierpumpe auf der Basis thermischer Aktoren. Bei Verwendung thermischer Aktoren wird das Aromenkonzentrat auf mikroskopischen "Kochplatten" verdampft (Heizelement). Desweiteren dienen die thermischen Aktoren zum Nachpumpen von Aromenkonzentrat. Bei Auswahl der Aromenkonzentrate ist auf eine rückstandsfreie Verdampfung zu achten.

Beim Start wird zuerst das Heizelement 15 nahe der Austrittsdüse 15 aktiviert, um die dort befindliche Flüssigkeit zu verdampfen und als Aromenwolke auszustoßen. Nach Leeren des Verdampfers wird durch Aktivierung von Heizelement 16 im Zulauf Flüssigkeit nachgepumpt. Nach Erkalten der Heizelemente wird neues Aromenkonzentrat durch die Kapillarkräfte nachgesaugt. Zur Dosierung der Aromenmenge kann wie bei der Ausführungsvariante mit dem Piezoelement der beschriebene Zyklus beliebig oft wiederholt werden. Die Aufheizung des Aromenkonzentrats kann durch Wiederstandsheizung, induktiv, auf Mikrowellenbasis etc. erfolgen.

Eine Mikrodosierpumpe auf der Basis thermischer Aktoren kann auch nach dem bekannten Prinzip der Tintenstrahldrucker arbeiten. In der Austragdüse wird im Abstand von der Düsenöffnung mittels eines Heizelments eine Dampfblase erzeugt, die einen Flüssigkeitstropfen, hier des Aromenkonzentrats, ins Freie befördert. Der Flüssigkeitstropfen kann wiederum zerstäubt oder verdampft werden.

Figur 11 zeigt den Austrag gasförmigen Aromenkonzentrats mit Piezoventil-Steuerung. Das Aromenkonzentrat kann dabei gasförmig unter Druck gespeichert sein oder es kann mittels Heizelement am Ausgang eines Aromenflüssigspeichers gasförmiges Aromenkonzentrat kurzfristig erzeugt werden. Ein Piezoelement 17 verschließt im Ruhezustand die Austragsöffnung 18 mittels eines zugeordneten Dichtelements 19. Bei Anlegen eines Spannungsimpulses schnellt das Piezoelement 17 nach oben und öffnet die Austragöffnung 18, so dass das unter Druck stehende Aromenkonzentrat ausströmt. Durch mehrfaches Pulsen kann die Aromenmenge dosiert werden. Die Generierung der Aromenwolke kann wie zuvor mittels mechanischer Zerstäubungsdüse, auf Ultraschall- oder elektrostatischer Basis etc. erfolgen.

In Figur 12 ist schematisch dargestellt, wie durch Synchronisieren des Duftausstoßes mit dem Atmungsvorgang des Nutzers die Duft-/Aromenwolke und damit auch die Duftspeichermenge gering gehalten werden kann. Dem Duftemitter 29 ist ein Beschleunigungssensor 26 mit Filter 27 und Auswerteinheit 28 zugeordnet, welche die aktuelle Atmungsfrequenz des Nutzers analysiert und somit den nächsten Einatmungsvorgang vorausberechnen kann. Unter Einberechnung der Duftfreisetzungszeit und der durchschnittlichen Strömungszeit des Duftes zur Nase wird ein Wait & Go Signal an den Duftemitter 29 weitergegeben. Der Duft wird somit nur freigesetzt, wenn er eine Chance hat, die Nase bei einem Einatmungsvorgang zu treffen. Es ergeben sich folgende Vorteile:
- die zum Erzeugen eines Dufteindrucks erforderliche Duftstoffmenge sinkt um etwa den Faktor 3,
- das Risiko der ungewollten Beduftung des Nachbarn sinkt,
- der Energieverbrauch des Duftemitters sinkt,
- die Atemanalyse kann auch genutzt werden, um zu erkennen, ob der Duft beim Träger angekommen ist, um die Dosierung danach anzupassen.

## Patentansprüche

1. Gerät zur Abgabe von Düften synchron zu einer Signal- oder Taktgebereinheit, mit einem Aromenspeicher, einer Steuereinheit zur Ansteuerung des Aromenspeichers und mit einer Austrageinheit zur Generierung und zum Austragen einer Duft- bzw. Aromenwolke aus dem Aromenspeicher, wobei
a) das Gerät als eine miniaturisierte mobile Einheit mit eigener Stromversorgung zum Tragen am Körper des Nutzers bzw. zur Montage, zum Anklemmen oder Anklipsen im Bereich der Körperwärme des Nutzers, ausgebildet ist,
b) der Steuereinheit ein Empfangsmodul zugeordnet ist zum Empfang von Steuersignalen einer externen Signal- bzw. Taktgebereinheit,
c) und die Austrageinheit ausgebildet ist, um die angesteuerten, in dem Gerät gespeicherten Duftstoffe ohne Zuhilfenahme eines Trägergases durch Zerstäuben und/oder durch Verdampfen dieser Duftstoffe aus dem Aromenspeicher auszutragen.

2. Gerät nach Anspruch 1,
**dadurch gekennzeichnet, dass** die Austrageinheit eine Einrichtung zur Feststellung der Atmungsfrequenz des Nutzers aufweist, um die angesteuerten, in dem Gerät gespeicherten Duftstoffe synchron zum Atmungsvorgang des Nutzers auszutragen.

3. Gerät nach Anspruch 1 oder 2,
**dadurch gekennzeichnet, dass** der Aromenspeicher als ein durch die Steuereinheit ansteuerbarer bzw. Mikrochip oder als Chipkarte bzw. Duftchip mit Duftstoff-Speicherplätzen ausgebildet ist,

4. Gerät nach Anspruch 3,
**dadurch gekennzeichnet, dass** der Duftchip mit den Duftstoffe-Speicherplätzen als ein Auswechselteil ausgebildet ist.

5. Gerät nach Anspruch 4,
**dadurch gekennzeichnet, dass** der Duftchip einen Träger aufweist, in bzw. auf dem die Duftstoffe flüssig, gel- oder gasformig oder als Feststoff angeordnet sind.

6. Gerät nach Anspruch 4,
**dadurch gekennzeichnet, dass** der Duftchip einen Träger mit einer Anordnung von Porösstoffen aufweist, in bzw. an dem die Duftstoffe flüssig, gelförmig oder in Form von Feststoffablagerungen gebunden sind.

7. Gerät nach Anspruch 4,
**dadurch gekennzeichnet, dass** der Duftchip einen Träger mit einer Anordnung von Mikrotanks aufweist, in denen die Duftstoffe flüssig, gelförmig oder gasförmig aufgenommen sind und die durch eine Schutzschicht abgedeckt sind.

8. Gerät nach einem der Ansprüche 5 bis 7,
**dadurch gekennzeichnet, dass** die Duftstoff-Speicherplätze ein Reagenz enthalten ist zur Auslösung einer z. B. exothermen Reaktion unter definierten Bedingungen.

9. Gerät nach einem der Ansprüche 3 bis 8,
**dadurch gekennzeichnet, dass** in dem Gerät den Duftstoff-Speicherplätzen jeweils ein von der Steuereinheit ansteuerbares Element zum Austragen von Duftstoff zugeordnet ist.

10. Gerät nach Anspruch 9,
**dadurch gekennzeichnet, dass** in dem Gerät den Duftstoffe-Speicherplätzen jeweils ein von der Steuereinheit ansteuerbares Element zum thermischen und/oder elektro-chemischen Austragen von Duftstoff zugeordnet ist.

11. Gerät nach Anspruch 9 in Verbindung mit Anspruch 7,
**dadurch gekennzeichnet, dass** in dem Gerät den Duftstoffe-Speicherplätzen jeweils ein von der Steuereinheit ansteuerbares Element zum Aufbrechen der Mikrotanks zugeordnet ist.

12. Gerät nach Anspruch 9 in Verbindung mit Anspruch 8,
**dadurch gekennzeichnet, dass** in dem Gerät den Duftstoffe-Speicherplätzen jeweils ein Element zur Schaffung der definierten Bedingungen zur Auslösung der z.B. exothermen Reaktion für das Reagenz zugeordnet ist.

13. Gerät nach Anspruch 1 oder 2,
**dadurch gekennzeichnet, daß** die Duftstoffe flüssig in einer Aromenvorratskartusche gespeichert sind und die Austrageinheit ausgebildet ist die gespeicherten Duftstoffe mittels einer Mikrodosierpumpe aus zutragen.

14. Gerät nach Anspruch 13,
**dadurch gekennzeichnet, daß** eine Mikrodosierpumpe auf der Basis piezoelektrischer Aktoren vorgesehen ist.

15. Gerät nach Anspruch 13,
**dadurch gekennzeichnet, daß** eine Mikrodosierpumpe auf der Basis thermischer Aktoren vorgesehen ist.

16. Gerät nach Anspruch 1 oder 2,
**dadurch gekennzeichnet, daß** die Duftstoffe flüssig oder gasförmig in dem Aromenspeicher gespeichert sind und die Austrageinheit ausgebildet ist, gasförmiges Aromenkonzentrat mit Piezoventil-Steuerung auszutragen.

17. Gerät nach einem der Ansprüche 13-16,
**dadurch gekennzeichnet, daß** die Austrageinheit eine mechanische Zerstäubungsdüse aufweist.

18. Gerät nach einem der Ausprüche 13-16,
**dadurch gekennzeichnet, daß** die Austrageinheit mit einer Ultraschall-Zerstäubungseinrichtung ausgerüstet ist.

19. Gerät nach einem der Ausprüche 13-16,
**dadurch gekennzeichnet, daß** die Austrageinheit mit einer elektrostatischen Zerstäubungseinrichtung ausgerüstet ist.

20. Gerät nach einem der Ausprüche 13-16,
**dadurch gekennzeichnet, daß** der Austrageinheit ein Mikroheizelement zum Verdampfen der ausgetragenen Duftstoffe zugeordnet ist.

21. Gerät nach einem der Ausprüche 13-16,
**dadurch gekennzeichnet, daß** der Austrageinheit eine Mikrowelleneinheit zum Verdampfen der ausgetragenen Duftstoffe zugeordnet ist.

22. Gerät nach einem der vorangehenden Ansprüche,
**gekennzeichnet durch** ein kleines Gebläse zur Unterstützung der Aufwärtsbewegung der ausgetragenen Duft- bzw. Aromenwolke aufgrund natürlicher Konvektion hervergerufen **durch** Körperwärme.

23. Gerät nach einem der vorangehenden Ansprüche,
**gekennzeichnet durch** eine Heizeinrichtung zur Entfaltung der ausgetragenen Duft- bzw. Aromenwolke.

24. Aromenspeicher bzw. Duftchip insbesondere zur Verwendung mit einem Gerät zur Abgabe von Düften nach einem der Ansprüche 1 bis 12,
**gekennzeichnet durch** einen Träger (20), in bzw. auf dem die Duftstoffe flüssig, gel- oder gasförmig oder als Feststoff angeordnet sind.

25. Aromenspeicher bzw. Duftchip nach Anspruch 24,
**gekennzeichnet durch** eine Anordnung von Porösstoffen (21), in bzw. an den die Duftstoffe flüssig, gelförmig oder in Form von Feststoffablagerungen gebunden sind.

26. Aromenspeicher bzw. Duftchip nach Anspruch 25,
**gekennzeichnet durch** einen Träger (20) in Form einer Harz/Kunststoff- oder Pappefolie mit einer Anordnung von Vertiefungen/Löchern, in denen die Porösstoffe (21) aufgenommen sind.

27. Aromenspeicher bzw. Duftchip nach Anspruch 26,
**gekennzeichnet durch** eine den Porösstoff (21) vom Trägermaterial (20) isolierende Metall- oder metallbedampfte Kunststoffschale (20',20").

28. Aromenspeicher bzw. Duftchip nach Anspruch 27,
**gekennzeichnet durch** eine elektrische Trennschicht auf der Unterseite der Trägerfolie (20).

29. Aromenspeicher bzw. Duftchip nach Anspruch 26,
**dadurch gekennzeichnet, dass** die Porösstoffe in eine Silikonoder Kunstharzmasse eingebettet sind.

30. Aromenspeicher bzw. Duftchip nach einem der Ansprüche 25 bis 29,
**dadurch gekennzeichnet, dass** die duftstoffgetränkten Porösstoffe (21) auf ihrer Oberseite z. B. mittels Wachs versiegelt sind.

31. Aromenspeicher bzw. Duftchip nach Anspruch 24,
**gekennzeichnet durch** eine Anordnung von Mikrotanks, in denen die Duftstoffe flüssig, gelförmig oder gasförmig aufgenommen sind, und **durch** eine die Mikrotanks verschließende Schutzschicht.

32. Aromenspeicher bzw. Duftchip nach einem der Ansprüche 24 bis 31,
**dadurch gekennzeichnet, dass** der Anordnung von Duftstoffen ein Reagenz zugeordnet ist zur Auslösung einer z. B. exothermen Reaktion unter definierten Bedingungen.

## Claims

1. An appliance for dispensing scents in sync with a signal or pulse generating unit, having an aroma store, a control unit for controlling the aroma store, and a discharge unit for generating and discharging a scent/aroma cloud from the aroma store, wherein
a) the appliance is embodied as a miniaturized mobile unit with its own power supply to be worn on the body or to be installed, clamped or clipped in the vicinity of the users body heat,
b) a receiver module is assigned to the control unit to receive control signals from an external signal or pulse generating unit,
c) and the discharge unit is adapted to discharge the controlled scent substances stored in the appliance from the aroma store without the assistance of a carrier gas by atomizing and/or vaporizing these scent substances.

2. The appliance of claim 1, wherein the discharge unit has an installation to determine the breathing cycle of the user to discharge the controlled scent substances stored in the appliance in sync with the user's respiratory process.

3. The appliance of claim 1 or 2, wherein the aroma store is embodied as a microchip that can be controlled by the control unit or as a chip card/scent chip having sent substance storage locations.

4. The appliance of claim 3, wherein the scent chip having the scent substance storage locations is embodied as a replaceable part.

5. The appliance of claim 4, wherein the scent chip has a carrier in or on which the scent substances are disposed in the form of liquids, gels, gases, or solids.

6. The appliance of claim 4, wherein the scent chip has a carrier with an arrangement of porous substances in or on which the scent substances are attached in the form of liquids, gels, or solid deposits.

7. The appliance of claim 4, wherein the scent chip has a carrier with an arrangement of microtanks that hold the scent substances in liquid, gel, or gaseous form and that are covered by a protective layer.

8. The appliance of claims 5 to 7, wherein a reagent is assigned to the scent substance storage locations in order to initiate a reaction, for example an exothermic reaction, under defined conditions.

9. The appliance of one of claims 3 to 8, wherein in the appliance one element that can be controlled by the control unit and that is used to discharge scent substance is assigned to each scent substance storage location.

10. The appliance of claim 9, wherein in the appliance one element that can be controlled by the control unit and that is used to discharge scent substance by thermal and/or electrochemical means is assigned to each scent substance storage location.

11. The appliance of claim 9 in combination with claim 7, wherein in the appliance one element that can be controlled by the control unit and that is used to break open the microtank is assigned to each scent substance storage location.

12. The appliance of claim 9 in combination with claim 8, wherein in the appliance one element that is used to establish the defined conditions, to initiate for example the exothermic reaction, for the reagent is assigned to each scent substance storage location.

13. The appliance of claim 1 or 2, wherein the scent substances are stored in liquid form in an aroma reservoir cartridge, and the discharge unit is adapted to discharge the stored scent substances by means of a micro metering pump.

14. The appliance of claim 13, wherein a micrometering pump utilizing piezoelectric actuators is provided.

15. The appliance of claim 13, wherein a micrometering pump utilizing thermal actuators is provided.

16. The appliance of claim 1 or 2, wherein the scent substances are stored in liquid or gaseous form in the aroma store, and the discharge unit is adapted to discharge gaseous aroma concentrate using a piezo valve controller.

17. The appliance of one of claims 13 to 16, wherein the discharge unit has a mechanical atomizing nozzle.

18. The appliance of one claims 13 to 16, wherein the discharge unit is equipped with an ultrasonic atomizing device.

19. The appliance of one of claims 13 to 16, wherein the discharge unit is equipped with an electrostatic atomizing device.

20. The appliance of one of claims 13 to 16, wherein a microheating element for vaporizing the discharged scent substances is assigned to the discharge unit.

21. The appliance of one of claims 13 to 16, wherein a microwave unit for vaporizing the discharged scent substances is assigned to the discharge unit.

22. The appliance of one of the above claims, **characterized by** a small blower to assist the upward movement of the discharged scent or aroma cloud that occurs due to natural convention caused by body heat.

23. The appliance of one of the above claims, **characterized by** a heater to enhance the discharged scent or aroma cloud.

24. An aroma store or scent chip used in particular with an appliance to discharge scents as recited in one of claims 1 to 12, **characterized by** a carrier (20) in or on which the scent substances are disposed in liquid, gel, gaseous or solid form.

25. The aroma store or scent chip of claim 24, **characterized by** an arrangement of porous substances (21) in or on which the scent substances are attached in the form of a liquid, gel, or solid deposits.

26. The aroma store or scent chip of claim 25, **characterized by** a carrier (20) in the form of a resin/plastic or cardboard sheet having an arrangement of depressions/holes holding the porous substances (21).

27. The aroma store or scent chip of claim 26, **characterized by** metal or metal vapor-deposited plastic shell (20', 20") that insulates the porous substance (21) from the carrier material (20).

28. The aroma store or scent chip of claim 27, **characterized by** an electrical insulating layer on the underside of the carrier sheet (20).

29. The aroma store or scent chip of claim 26, wherein the porous substances are embedded in a silicon or plastic resin compound.

30. The aroma store or scent chip of one of claims 25 to 29, wherein the scent substance-saturated porous substances (21) are sealed on their upper side, for example by means of a wax.

31. The aroma store or scent chip of claim 24, **characterized by** an arrangement of microtanks in which the scent substances are held in liquid, gel or gaseous form and by a protective layer that seals the microtanks.

32. The aroma store or scent chip of one of claims 24 to 31, wherein a reagent is assigned to the arrangement of scent substances to initiate a reaction, for example an exothermic reaction, under defined conditions.

## Revendications

1. Appareil pour diffuser des senteurs de manière synchronisée avec une unité émettant un signal ou une cadence avec un réservoir d'arômes, une unité d' commande pour commander le réservoir d'arômes et avec une unité de diffusion pour produire et pour diffuser un nuage de senteur ou d'arômes du réservoir d'arômes, dans lequel appareil :
a) l'appareil est formé d'une unité mobile miniaturisée avec sa propre alimentation en courant pour être porté sur le corps de l'utilisateur ou pour être monté, pincé ou clipsé dans la zone de la chaleur corporelle de l'utilisateur,
b) un module de réception est associé à l'unité de commande pour recevoir des signaux de commande d'une unité externe émettant un signal ou une cadence,
c) l'unité de diffusion est formée pour diffuser les senteurs commandés et stockées dans l'appareil sans l'aide d'un gaz porteur par pulvérisation et / ou évaporation de ces senteurs hors du réservoir d'arômes .

2. Appareil selon la revendication 1, **caractérisé en ce que** l'unité de diffusion présente un dispositif pour établir la fréquence de respiration de l'utilisateur afin de diffuser les senteurs commandées et stockées dans l'appareil de manière synchronisée par rapport au processus d'inspiration de l'utilisateur.

3. Appareil selon la revendication 1 ou 2, **caractérisé en ce que** le réservoir d'arômes est formé en tant que micro-plaquette ou en tant que carte de plaquettes ou de plaquette de senteurs , pouvant être commandées par une unité de commande, avec des places de réservoir de senteurs.

4. Appareil selon la revendication 3, **caractérisé en ce que** la plaquette de senteurs est formée avec les places de réservoir de senteurs en tant que pièce de rechange.

5. Appareil selon la revendication 4, **caractérisé en ce que** la plaquette de senteurs présente un support, notamment sur lequel sont disposés, les senteurs de manière liquide, sous forme de gel ou de gaz ou en tant que matières solides.

6. Appareil selon la revendication 4, **caractérisé en ce que** la plaquette de senteurs présente un support avec une disposition de matériaux poreux, dans lequel, notamment sur lequel les senteurs sont liés de manière liquide, sous la forme d'un gel ou sous la forme de dépôts de matériaux solides.

7. Appareil selon la revendication 4, **caractérisé en ce que** la plaquette de senteurs présente un support avec une disposition de micro-réservoirs dans lesquels les matériaux de senteurs sont reçus de manière liquide, sous la forme de gel ou de gaz et recouverts par une couche de protection.

8. Appareil selon les revendications 5 à 7, **caractérisé en ce que** les places de réservoir de senteurs comprennent une substance pour déclencher une réaction, par exemple, exotherme selon des conditions définies.

9. Appareil selon les revendications 3 à 8, **caractérisé en ce que** dans l'appareil, on a associé à chaque fois aux places de réservoir de senteurs un élément commandé par l'unité de commande pour diffuser la senteur.

10. Appareil selon la revendication 9, **caractérisé en ce qu'**on a associé à chaque fois aux places de réservoir de senteurs un élément commandé par une unité de commande pour diffuser de manière thermique et / ou électrochimique des senteurs .

11. Appareil selon la revendication 9, en liaison avec la revendication 7, **caractérisé en ce qu'**on a associé à chaque fois aux places de réservoir de senteurs , un élément commandé par l'unité de commande pour casser le micro-réservoir.

12. Appareil selon la revendication 9, en liaison avec la revendication 7, **caractérisé en ce qu'**on a associé à chaque fois aux places de réservoir de senteurs un élément pour réaliser les conditions définies, pour déclencher la réaction, par exemple, exotherme pour la substance .

13. Appareil selon la revendication 1 ou 2, **caractérisé en ce que** les senteurs sont stockées de manière liquide dans une cartouche de stockage d'arômes et **en ce que** l'unité de diffusion est formée afin de diffuser les senteurs stockées au moyen une pompe à microdosage.

14. Appareil selon la revendication 3, **caractérisé en ce qu'**une pompe à micro-dosage est prévue sur la base d'agents piézoélectriques.

15. Appareil selon la revendication 3, **caractérisé en ce qu'**une pompe à micro-dosage est prévue sur la base d'éléments thermiques.

16. Appareil selon la revendication 1 ou 2, **caractérisé en ce que** les senteurs sont stockées de manière liquide ou sous forme de gaz dans le réservoir d'arômes et **en ce que** l'unité de diffusion est formée pour diffuser le concentré d'arômes sous forme de gaz par une commande à piézo-valve.

17. Appareil selon une des revendications 13-16, **caractérisé en ce que** l'unité de diffusion comporte une buse de pulvérisation mécanique.

18. Appareil selon une des revendications 13-16, **caractérisé en ce que** l'unité de diffusion comporte un dispositif de pulvérisation à ultrasons.

19. Appareil selon une des revendications 13-16, **caractérisé en ce que** l'unité de diffusion est équipée d'un dispositif de pulvérisation électrostatique.

20. Appareil selon une des revendications 13-16, **caractérisé en ce qu'**on associe à l'unité de diffusion un micro-élément chauffant pour évaporer les senteurs diffusées .

21. Appareil selon une des revendications 13-16, **caractérisé en ce qu'**on associe à l'unité de diffusion une unité à micro-ondes pour évaporer les senteurs diffusées.

22. Appareil selon une des revendications précédentes, **caractérisé par** un petit ventilateur destiné à assister le mouvements vers l'avant du nuage de senteur ou d'arômes diffusés sur la base d'une convection naturelle générée par la chaleur humaine.

23. Appareil selon une des revendications précédentes, **caractérisé par** un dispositif de chauffage destiné à diffuser le nuage de senteurs ou d'arômes émis.

24. Réservoir d'arômes ou plaquette de senteurs , notamment pour être utilisé avec un appareil pour diffuser de senteurs selon les revendications 1 à 13, **caractérisé par** un support (20) dans lequel ou sur lequel les senteurs sont disposées de manière liquide, sous forme de gel ou de gaz ou sous forme de matières solides.

25. Réservoir d'arômes ou plaquette de senteurs selon la revendication 4, **caractérisé par** une disposition de matériaux poreux (21) dans lesquels ou auxquels les senteurs sont liées de manière liquide, sous la forme de gel ou sous la forme de dépôts de matériaux solides .

26. Réservoir d'arômes ou plaquette de senteurs selon la revendication 5, **caractérisé par** un support (20) ayant la forme d'une feuille en résine / matière plastique ou d'une feuille en carton avec une disposition de trous / renfoncements dans lesquels les matériaux poreux (21) sont reçus ).

27. Réservoir d'arômes ou plaquette de senteurs selon la revendication 6, **caractérisé par** une enveloppe en métal, en matière plastique (20', 20") traité au métal isolant le matériaux poreux (21) du matériau support (20).

28. Réservoir d'arômes ou plaque de senteurs selon la revendication 27, **caractérisé par** une couche de séparation électrique sur le coté inférieur de la feuille de support (20).

29. Réservoir d'arômes ou plaque de senteurs selon la revendication 26, **caractérisé en ce que** les matériaux poreux sont incorporés dans une masse en silicone ou en résine plastique .

30. Réservoir d'arômes ou plaquette de senteurs selon la revendication 5 à 29, **caractérisé en ce que** les matériaux poreux (21) imbibés de senteurs sont scellés sur leur coté supérieur, par exemple, au moyen de cire.

31. Réservoir d'arômes ou plaquette de senteurs selon la revendication 4, **caractérisé par** une disposition de micro-réservoirs dans lesquels les senteurs sont reçues de manière liquide, sous la forme de gel ou de gaz et **caractérisé par** une couche de protection fermant les micro-réservoirs.

32. Réservoir d'arômes ou plaquette de senteurs selon une des revendications 4 à 1, **caractérisé en ce qu'**une substance est associée à la disposition de senteurs pour déclencher une réaction, par exemple, exotherme selon des conditions définies.
